(19) Europäisches Patentamt
European Patent Office
Office européen des brevets

(11) EP 1 506 193 B3

(12) **NEUE EUROPÄISCHE PATENTSCHRIFT**
Nach dem Beschränkungsverfahren (B3-1)

(45) Hinweis auf die Patenterteilung:
**21.06.2006 Patentblatt 2006/25**

(45) Veröffentlichungstag und Bekanntmachung des Hinweises auf die Entscheidung über den Antrag auf Beschränkung:
**B3-1 03.12.2025 Patentblatt 2025/49**

(21) Anmeldenummer: **03727359.6**

(22) Anmeldetag: **25.04.2003**

(51) Internationale Patentklassifikation (IPC):
***C07D 471/04*** *(2006.01)* ***A61K 31/506*** *(2006.01)*
***A61P 9/02*** *(2006.01)*

(52) Gemeinsame Patentklassifikation (CPC):
(C-Sets verfügbar)
**C07D 471/04; A61K 31/506; A61K 45/06; A61P 9/00; A61P 9/02; A61P 9/10; A61P 9/12; A61P 13/00; A61P 15/00; A61P 15/08; A61P 15/10; A61P 19/10; A61P 25/00; A61P 25/16; A61P 25/24;**
(Forts.)

(86) Internationale Anmeldenummer:
**PCT/EP2003/004304**

(87) Internationale Veröffentlichungsnummer:
**WO 2003/095451 (20.11.2003 Gazette 2003/47)**

(54) **CARBAMAT-SUBSTITUIERTE PYRAZOLOPYRIDINE**

CARBAMATE-SUBSTITUTED PYRAZOLOPYRIDINES

PYRAZOLOPYRIDINES A SUBSTITUTION CARBAMATE

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **08.05.2002 DE 10220570**

(43) Veröffentlichungstag der Anmeldung:
**16.02.2005 Patentblatt 2005/07**

(73) Patentinhaber: **Adverio Pharma GmbH**
**51373 Leverkusen (DE)**

(72) Erfinder:
- **ALONSO-ALIJA, Cristina**
  **42781 Haan (DE)**
- **BISCHOFF, Erwin**
  **42115 Wuppertal (DE)**
- **MÜNTER, Klaus**
  **42489 Wülfrath (DE)**
- **STASCH, Johannes-Peter**
  **42651 Solingen (DE)**
- **STAHL, Elke**
  **51467 Bergisch Gladbach (DE)**
- **WEIGAND, Stefan**
  **42115 Wuppertal (DE)**
- **FEURER, Achim**
  **69259 Wilhelmsfeld (DE)**

(74) Vertreter: **Hoffmann Eitle**
**Patent- und Rechtsanwälte PartmbB**
**Arabellastraße 30**
**81925 München (DE)**

(56) Entgegenhaltungen:
**WO-A1-00/06567** **WO-A1-02/092596**
**WO-A1-02/42299** **WO-A1-02/42300**
**WO-A1-02/42301** **WO-A1-02/42302**
**WO-A1-03/004503** **DE-A1- 19 834 044**
**DE-A1- 19 846 514**

Bemerkungen:
Die Akte enthält technische Angaben, die nach dem Eingang der Anmeldung eingereicht wurden und die nicht in dieser Patentschrift enthalten sind.

EP 1 506 193 B3

(52) Gemeinsame Patentklassifikation (CPC): (Forts.)
**A61P 25/28; A61P 27/06**

C-Sets
**A61K 31/506, A61K 2300/00**

## Beschreibung

Die vorliegende Erfindung betrifft Verbindungen, die die lösliche Guanylatcyclase stimulieren, ihre Herstellung und ihre Verwendung als Arzneimittel, insbesondere als Arzneimittel zur Behandlung von Herz-Kreislauf-Erkrankungen und/oder sexueller Dysfunktion.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

**[0003]** Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/eGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall, sexueller Dysfunktion und Myokardinfarkt führen kann.

Eine auf die Beeinflussung des cGMP-Signalweges in Organismen abzielende NOunabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylateyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Brit. J. Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587) sowie verschiedene substituierte Pyrazolderivate (WO 98/16223).

Weiterhin sind in WO 98/16507, WO 98/23619, WO 00/06567, WO 00/06568, WO 00/06569, WO 00/21954 WO 02/42299, WO 02/42300, WO 02/42301, WO 02/42302, WO 02/092596 und WO 03/004503 Pyrazolopyridinderivate als Stimulatoren der löslichen Guanylatcyclase beschrieben. Unter anderem sind dort auch Pyrazolopyridine beschrieben, die einen Pyrimidinrest in 3-Position aufweisen. Derartige Verbindungen weisen eine sehr hohe in vitro Aktivität bezüglich der Stimulation der löslichen Guanylatcyclase auf. Allerdings zeigte es sich, dass diese Verbindungen hinsichtlich ihrer in vivo-Eigenschaften wie beispielsweise ihrem Verhalten in der Leber, ihrem pharmakokinetischen Verhalten, ihrer Dosis-Wirkungsbeziehung oder ihrem Metabolisierungsweg Nachteile aufweisen.

Es war daher die Aufgabe der vorliegenden Erfindung, weitere Pyrazolopyridinderivate bereitzustellen, welche als Stimulatoren der löslichen Guanylatcyclase wirken, aber nicht die vorstehend aufgeführten Nachteile der Verbindungen aus dem Stand der Technik aufweisen.

Diese Aufgabe wird durch die erfindungsgemäßen Verbindungen gemäß Anspruch 1 gelöst. Diese neue Klasse von Pyrazolopyridinderivaten zeichnet sich durch einen Pyrimidinrest in 3-Position aus, der ein bestimmtes Substitutionsmuster aufweist, nämlich einen Carbamatrest in 5-Position des Pyrimidinrings sowie eine Aminogruppe in 4-Position des Pyrimidinrings.

Im einzelnen betrifft die vorliegende Erfindung
die Verbindung Methyl-4,6-diamino-2-[1-(2-fluorbenzyl)-1H-pyrazolo[3,4-b]pyridin-3-yl]-5-pyrimidinyl(methyl)carbamat aus Beispiel 8:

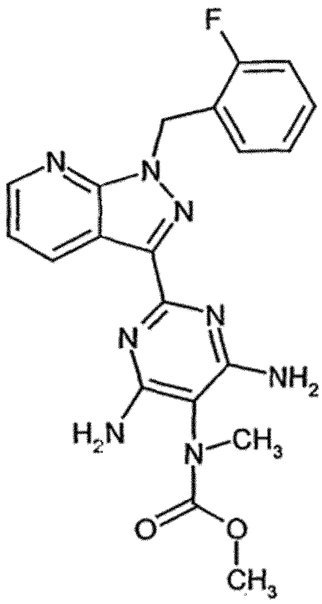

Die erfindungsgemäße Verbindung kann auch in Form ihrer Salze vorliegen. Imallgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindung können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindung sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäße Verbindung kann in tautomeren Formen vorliegen. Dies ist dem Fachmann bekannt, und derartige Formen sind ebenfalls vom Umfang der Erfindung umfasst.

Weiterhin kann die erfindungsgemäße Verbindung in Form ihrer möglichen Hydrate vorkommen.

Gegenstand der vorliegenden Erfindung ist auch ein Verfahren zur Herstellung der erfindungsgemäßen Verbindung. Diese kann hergestellt werden

[A] durch Umsetzung von Verbindungen der Formel (Ia)

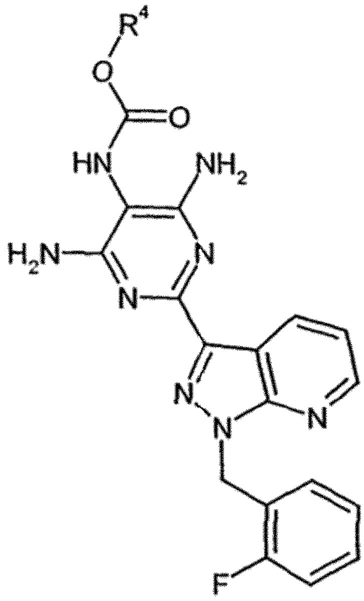

(Ia),

worin

$R^4$ Methyl ist,
mit Verbindungen der Formel (II)

$R^3$-$X^1$ (II),

worin
$R^3$ Methyl ist und
$X^1$ für eine Abgangsgruppe wie beispielsweise Halogen, bevorzugt Iod, oder Mesylat steht,
gegebenenfalls in einem organischen Lösungsmittel unter Kühlung zur Verbindung des Beispiels 8.

[B] Die vorliegende Offenbarung betrifft auch die Umsetzung der Verbindung der Formel (III)

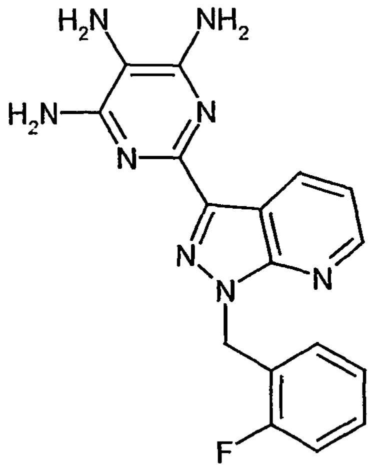

(III),

mit Verbindungen der Formel (IV)

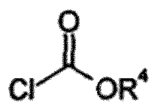

(IV),

worin

$R^4$ für ($C_1$-$C_6$)-Alkyl steht,,
gegebenenfalls in einem organischen Lösungsmittel zu Verbindungen der Formel (Ia), oder

[C] durch Umsetzung der Verbindung der Formel (V)

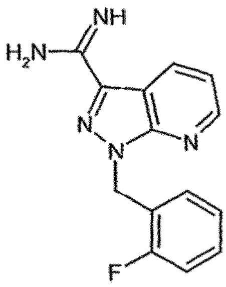

(V)

mit Verbindungen der Formel (VI)

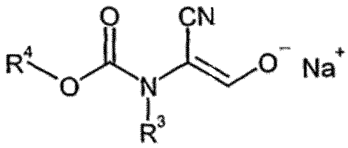

(VI),

worin
$R^3$ für Wasserstoff oder ($C_1$-$C_4$)-Alkyl steht, und $R^4$ wie vorstehend definiert ist,
gegebenenfalls in einem organischen Lösungsmittel unter Erhitzen zu Verbindungen der Formel (Ib)

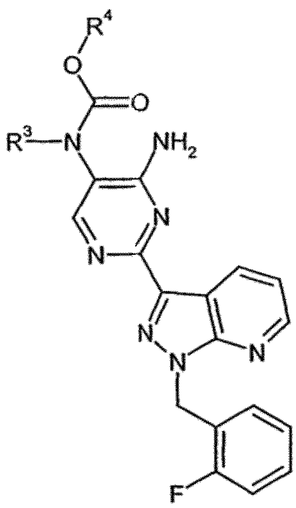

(Ib),

worin
$R^3$ und $R^4$ wie vorstehend definiert sind.

Die Verbindungen der Formel (II) und (IV) sind kommerziell erhältlich, literasturbekannt oder können auf dem Fachmann bekannte Weise dargestellt werden.

Die Verbindung der Formel (III) lässt sich gemäß folgendem Reaktionsschema herstellen:

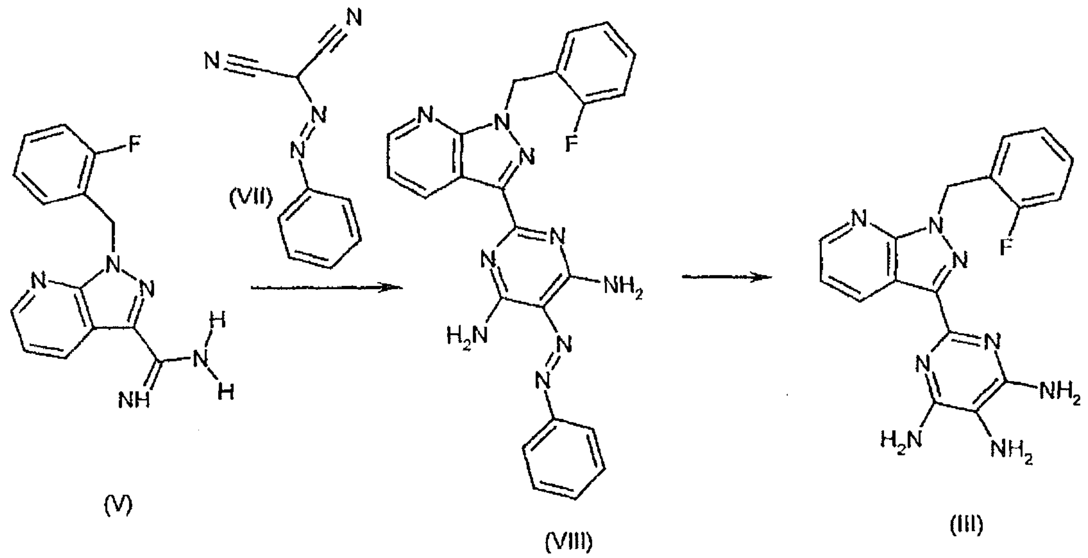

(V) (VII) (VIII) (III)

Verbindung (III) ist in einer zweistufigen Synthese durch Umsetzung von Verbindung (V) mit Verbindung (VII) zu Verbindung (VIII) entsprechend dem Verfahrensschritt [C] und anschließende Hydrierung der Verbindung (VIII) mit wässrigem Raney-Nickel erhältlich. Die Hydrierung kann in einem organischen Lösungsmittel, beispielsweise Dimethylformamid, vorzugsweise bei erhöhtem Druck, beispielsweise bei 50 bis 70 bar, vorzugsweise bei 65 bar, und Rühren der Reaktionslösung für mehrere Stunden, beispielsweise für 22 Stunden, bei erhöhter Temperatur, beispielsweise bei 40 bis 80°C, vorzugsweise bei 60°C bis 65°C, durchgeführt werden.

Die Verbindung (VII) kann analog L. F. Cavalieri, J. F. Tanker, A. Bendich, J. Am. Chem. Soc., 1949, 71, 533 dargestellt werden.

Die Verbindung (V) lässt sich gemäß folgendem Reaktionsschema herstellen:

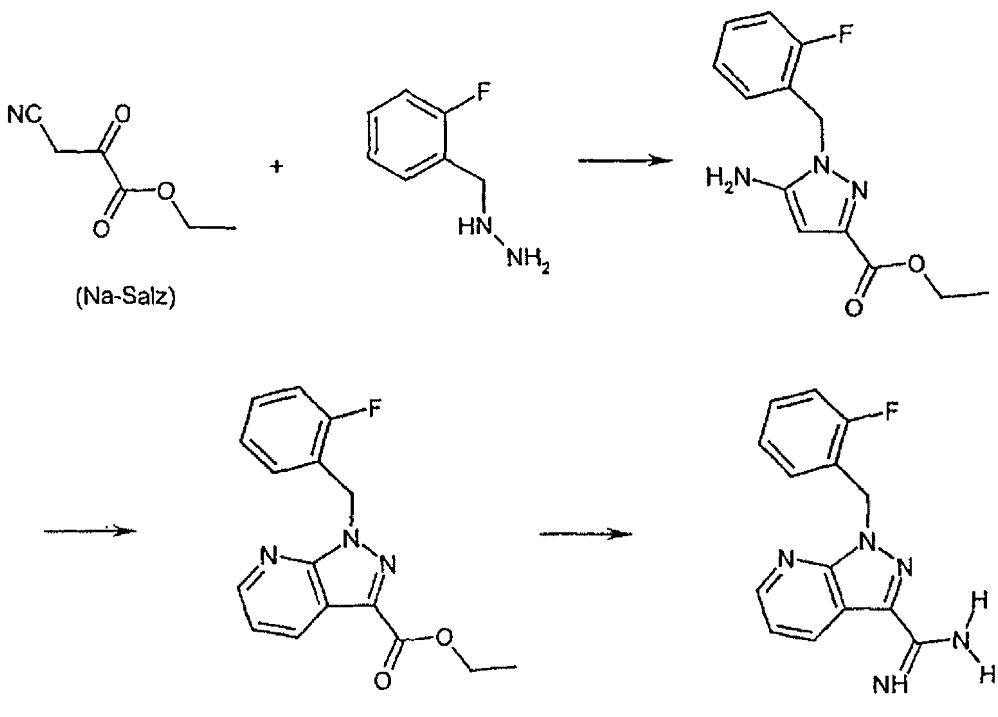

(Na-Salz)

Verbindung (V) ist in einer mehrstufigen Synthese aus dem literaturbekannten Natriumsalz des Cyanobrenztraubensäureethylesters (Borsche und Manteuffel, Liebigs. Ann. Chem. 1934, 512, 97) erhältlich. Durch dessen Umsetzung mit 2-Fluorbenzylhydrazin unter Erhitzen in einer Schutzgasatmosphäre in einem inerten Lösungsmittel wie Dioxan erhält man 5-Amino-1-(2-fluorbenzyl)-pyrazol-3-carbonsäureethylester, das durch Umsetzung mit Dimethylaminoacrolein im sauren Medium unter Schutzgasatmosphäre und Erhitzen zum entsprechenden Pyridinderivat cyclisiert werden kann. Dieses Pyridinderivat, 1-(2-Fluorbenzyl)-1H-pyrazolo[3,4-b]pyridin-3-carbonsäureethylester wird durch

eine mehrstufige Sequenz, bestehend aus Überführung des Esters mit Ammoniak in das entsprechende Amid, Dehydratisierung mit einem wasserentziehenden Mittel wie Trifluoressigsäureanhydrid zum entsprechenden Nitrilderivat, Umsetzung des Nitrilderivats mit Natriumethylat und abschließende Reaktion mit Ammoniumchlorid in die Verbindung (V) überführt.

Die Verbindungen der Formel (VI) können nach dem Fachmann bekannten Methoden aus den entsprechenden Carbamaten durch Reaktion mit Ameisensäureethylester synthetisiert werden. Die Carbamate können analog Q. Li. Chu, T. W. Daniel, A. Claibome, C. S. Cooper, C. M. Lee, J. Med. Chem. 39 (1996) 3070-3088 hergestellt werden.

Die Umsetzung der Verbindungen der Formeln (Ia) und (II) zur erfindungsgemäßen Verbindung kann durch Einsatz der Reaktanden in äquimolaren Mengen in einem organischen Lösungsmittel, beispielsweise Dimethylformamid oder Tetrahydrofuran, vorzugsweise in Gegenwart von 1 bis 2 Äquivalenten, vorzugsweise 1.1 bis 1.5 Äquivalenten einer Base, wie beispielsweise Natriumhydrid oder Natrium-*N,N*-bistrimethylsilylamid, vorzugsweise bei Normaldruck und Rühren der Reaktionslösung für wenige Stunden, beispielsweise für 1 Stunde, unter Kühlung, beispielsweise bei -10°C bis Raumtemperatur, vorzugsweise bei 0°C, durchgeführt werden.

Die Umsetzung der Verbindungen der Formeln (Iii) und (IV) zu den Verbindungen der Formel (ia) kann durch Einsatz der Reaktanden in äquimolaren Mengen in einem organischen Lösungsmittel, beispielsweise einer organischen Base, vorzugsweise Pyridin, vorzugsweise bei Normaldruck und Rühren der Reaktionslösung für mehrere Stunden, beispielsweise für 12 Stunden, bei 0°C bis Raumtemperatur, vorzugsweise bei Raumtemperatur, durchgeführt werden.

Die Umsetzung von Verbindungen der Formeln (V) und (VI) zu Verbindungen der Formel (Ib) bzw. von Verbindungen der Formeln (V) und (VII) zu Verbindungen der Formel (VIII) kann durch Einsatz der Reaktanden in äquimolaren Mengen beziehungsweise unter Verwendung der Verbindung der Formel (VI) im leichten Überschuss in einem organischen Lösungsmittel wie beispielsweise in einem Kohlenwasserstoff wie Toluol oder Xylol oder in NN-Dimethylformamid, vorzugsweise in Gegenwart von 2-3 Äquivalenten, vorzugsweise 2 Äquivalenten einer Base, wie beispielsweise Triethylamin oder Natriummethanolat, vorzugsweise bei Normaldruck und Rühren der Reaktionslösung für mehrere Stunden, beispielsweise für 9 Stunden, bei erhöhter Temperatur, beispielsweise bei 80-160°C, vorzugsweise bei 100-150°C, insbesondere bei 110°C, durchgeführt werden.

Die erfindungsgemäße Verbindung zeigt ein nicht vorhersehbares, wertvolles pharmakologisches Wirkspektrum.

Die erfindungsgemäße Verbindung bewirkt eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatzyklase und einen intrazellulären cGMP- Anstieg vermittelt. Außerdem verstärkt die erfindungsgemäße Verbindung die Wirkung von Substanzen, die den cGMP-Spiegel steigern, wie beispielsweise EDRF(Endothelium derived relaxingfactor), NO-Donatoren, Protoporphyrin IX, Arachidonsäure oder Phenylhydrazinderivaten.

Sie kann daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, peripheren Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektiler Dysfunktion, weiblicher sexueller Dysfunktion, Osteoporose, Glaukom, pulmonaler Hypertonie, Gastroparese und Inkontinenz eingesetzt werden.

Die erfindungsgemäße Verbindung ist auch zur Bekämpfung von Krankheiten im Zentralnervensystem geeignet, die durch Störungen des NO/cGMP-Systems gekennzeichnet sind. Insbesondere ist sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", Altersassoziierte Lern- und Gedächtnisstörungen, altersassoziierten Gedächtnisverlusten, vaskulärerDemenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimerscher Krankheit, Demenz mit Lewy-Körperchen, Demenz mit Degeneration der Frontallappen einschliesslich des Pick's Syndroms, Parkinsonscher Krankheit, progressiver nuclear palsy, Demenz mit corticobasaler Degeneration, Amyolateralsklerose (ALS), Huntingtonscher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignet sich die erfindungsgemäße Verbindung auch zur Regulation der cerebralen Durchblutung und stellen somit wirkungsvolle Mittel zur Bekämpfung von Migräne dar.

Auch eignet sich die erfindungsgemäße Verbindung zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso kann sie zur Bekämpfung von Schmerzzuständen eingesetzt werden.

Zudem besitzt die erfindungsgemäße Verbindung antiinflammatorische Wirkung und kann daher als entzündungshemmendes Mittel eingesetzt werden.

Darüber hinaus umfasst die vorliegende Erfindung auch die Kombination der erfindungsgemäßen Verbindung mit einem oder mehreren organischen Nitraten oder NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Beispielhaft und vorzugsweise seien genannt: Natriumnitroprussid, Nitroglycerin, Isosorbiddinitrat, Isosorbidmononitrat, Molsidomin und SIN-1.

Außerdem umfasst die vorliegende Erfindung auch die Kombination mit einer oder mehreren Verbindungen, die den Abbau von cyclischem Guanosinmonophosphat (cGMP) inhibieren. Dies sind vorzugsweise Inhibitoren der Phosphodiesterasen 1, 2 und 5; Nomenklatur nach Beavo und Reifsnyder (1990) TiPS 11 S. 150 bis 155. Besonders bevorzugt sind hierbei Inhibitoren der Phosphodiesterase 5 (PDE V-Hemmer), insbesondere eine der Verbindungen Sildenafil (Viagra™, EP-A 0 463 756, WO 94/28902), Vardenafil (WO 99/24433) oder Tadalafil (WO 95/19978). Durch diese Inhibitoren wird die Wirkung der erfindungsgemäßen Verbindungen potenziert und der gewünschte pharmakologische Effekt gesteigert.

## Biologische Untersuchungen

### Gefäßrelaxierende Wirkung in vitro

Kaninchen werden durch Nackenschlag betäubt und entblutet. Die Aorta wird entnommen, von anhaftendem Gewebe befreit, in 1,5 mm breite Ringe geteilt und einzeln unter einer Vorspannung in 5 ml-Organbädermit 37°C warmer, carbogen-begaster Krebs-Henseleit-Lösung folgender Zusammensetzung (mM) gebracht: NaCl: 119; KCl: 4,8; $CaCl_2$ x 2 $H_2O$: 1; $MgSO_4$ x 7 $H_2O$: 1,4; $KH_2PO_4$: 1,2; $NaHCO_3$: 25; Glucose: 10. Die Kontraktionskraft wird mit Statham UC2-Zellen erfasst, verstärkt und über A/D-Wandler(DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung untersucht und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50 % zu reduzieren ($IC_{50}$). Das Standardapplikationsvolumen beträgt 5 μl, der DMSO-Anteil in der Badlösung entspricht 0,1 %.

Der $IC_{50}$-Wert für die Verbindung aus Beispiel 1 (Bezugsbeispiel) beträgt 670 nM, der entsprechende Wert für die Verbindung aus Beispiel 8 beträgt 500 nM.

### Kaninchen-Modell

Adulte, männliche Chinchilla-Kaninchen mit einem Gewicht von 3 - 5 kg werden nach Lieferung mehrere Tage in Einzelhaltung adaptiert. Sie haben freien Zugang zu Wasser und können zwei Stunden pro Tag Futter zu sich nehmen. Die Tiere werden in einem 10/14 Stunden Tag-Nacht Rhythmus gehalten (Licht an, ab 8.00 Uhr), die Raumtemperatur beträgt 22 - 24°C.

Pro Behandlungsgruppe werden drei bis sechs Tiere verwendet und direkt vor Versuchsbeginn gewogen. Für die i.v. Gabe werden die Substanzen in Transcutol (GATTEFOSSE GmbH) gelöst und im Verhältnis 3/7 mit einer 20%igen Cremophorlösung (Cremophor (BASF), Wasser) verdünnt. Es wird ein Volumen von 0,5 ml/kg in die Ohrvene injiziert. Wasserlösliche Substanzen werden in 0,9 % Kochsalzlösung injiziert.

Für die orale Gabe werden die Testsubstanzen in einer Mischung von Glycerin: Wasser: Polyethylenglycol 6: 10:9.69 gelöst und in einem Volumen von 1 ml/kg mit der Schlundsonde appliziert.

Unter Ruhebedingungen ist der Kaninchenpenis in der Schamregion nicht sichtbar und von der Penishaut vollständig bedeckt. Die Erektion wird gewertet, indem man die Länge des hervortretenden Penis mit einer Schiebelehre misst. Die Messung wird 5, 10, 15, 30, 45, 60 und120 Minuten nach Substanzgabe durchgeführt, nach oraler Gabe zusätzlich auch noch nach 3, 4, 5 und 6 Stunden. Die Tiere werden dazu jedes Mal aus dem Käfig geholt, am Nackenfell und den Hinterläufen festgehalten, auf den Rücken gedreht und gemessen. Entsprechende Lösungsmittelkontrollen werden durchgeführt. (vergleiche Literatur: E. Bischoff, K. Schneider, Int. J. of Impotence Res. 2001, 13, 230-235; E. Bischoff, U. Niewoehner, H. Haning, M. Es Sayed, T. Schenke, K. H. Schlemmer, The Journal of Urology, 2001, 165, 1316-1318; E. Bischoff, Int. J. Impotence Res. 2001, 13, 146-148).

Die minimale effektive Dosis der Verbindung aus Beispiel 8 beträgt bei oraler Gabe 0.03 mg/kg (unter gleichzeitiger Gabe von 0.2 mg/kg i. V. Natriumnitroprussid SNP).

Bestimmung pharmakokinetischer Kenngrößen nach intravenöser und oraler Gabe

Die zu untersuchende Substanz wird Tieren (z.B. Maus, Ratte, Hund) intravenös als Lösung appliziert, die orale Applikation erfolgt als Lösung oder Suspension über eine Schlundsonde. Nach Substanzgabe wird den Tieren zu festgelegten Zeitpunkten Blut entnommen, dieses wird heparinisiert und anschließend wird daraus durch Zentrifugation Plasma gewonnen. Die Substanz wird im Plasma über LC/MSMS analytisch quantifiziert. Aus den so ermittelten Plasmakonzentrations-Zeit-Verläufen werden die pharmakokinetischen Kenngrößen mittels eines validierten pharmakokinatischen Rechenprogramms berechnet.

Nach oraler Gabe von 0.3 und 1.0 mg/kg (als Lösung in Solutol:EtOH:Wasser 1:1:8) in der Ratte zeigt die Verbindung aus Beispiel 8 die folgenden Plasma Konzentrationen (AUC):

$$\text{AUCnorm (0.3 mg/kg)} = 0.326\ [\text{kg*h/L}]$$

$$\text{AUCnorm (1.0 mg/kg)} = 0.548\ [\text{kg*h/L}]$$

Inhibition von Cytochrom P450-Enzymen

Das Potential der Inhibition von P-450 Isoenzymen, die für den Metabolismus wichtig sind, wird automatisiert im 96-well Format untersucht. Hierbei werden zwei verschiedene Assays verwendet.

Bei dem auf Bildung von fluoreszierenden Metaboliten basierenden Assay werden rekombinante Enzyme (z.B. CYP1A2, 2C8, 2C9, 2C19, 2D6 oder 3A4) und im allgemeinen Fluorescein- oder Coumarin-Teilstrukturen enthaltene Substrate eingesetzt. Es werden jeweils eine Substratkonzentration und 8 Konzentrationen des potentiellen Inhibitors verwendet. Nach Inkubation mit dem jeweiligen rekombinanten CYP Enzym wird mittels Fluoreszenzreader das Ausmaß an fluoreszierenden Metaboliten im Vergleich zur Kontrolle (ohne Inhibitor) ermittelt und ein $IC_{50}$-Wert berechnet [Anal. Biochem. 248, 188 (1997)].

Beim 2. Assay werden als Enzymquelle humane Lebermikrosomen und als CYP Isoform-selektive Substrate Phenacetin (CYP1A2), Diclofenac (CYP2C9), Dextromethorphan (CYP2D6) und Midazolam (CYP3A4) verwendet. Die Bildung des jeweiligen Metaboliten wird mittels LC-MS/MS gemessen. Unter Annahme kompetitiver Inhibition werden aus der Verminderung der Metabolitenbildung im Vergleich zur Kontrolle $K_i$-Werte berechnet (1 Substrat-, 3 Inhibitorkonzentrationen).

Induktion von Cytochrom P450-Enzymen in humanen Leberzellkulturen

Zur Untersuchung des Nebenwirkungspotentialsder erfindungsgemäßen Substanzen bezüglich einer Induktion von Cytochrom P450-Enzymen werden primäre humane Hepatozyten mit einer Zelldichte von 2,5 x $10^5$ Zellen zwischen zwei Schichten von Collagen in 24 well-Mikrotiterplatten bei 37°C bei 5 % $CO_2$ 8 Tage kultiviert. Das Zelikuiturmedium wird täglich gewechselt.

Nach 48 Stunden in Kultur werden die Hepatozyten über 5 Tage in Doppelbestimmung mit unterschiedlichen Konzentrationen der Testsubstanzen im Vergleich mit den Induktoren Rifampicin (RIF; 50 µM), Omeprazol (OME; 100 µM) und Phenobarbital (PB; 2 mM) behandelt. Die Endkonzentrationen der Testsubstanzen liegen bei 0,01 - 10 µg/ml.

Von den Zellkulturen wird der induktive Effekt der Testsubstanzen auf die Cytochrom (CYP) P450-Enzyme 1A2, 2B6, 2C19 und 3A4 durch Zugabe der Substrate 7-Ethoxyresorufin (CYP1A2), [$^{14}$C]-S-Mephenytoin (CYP2B6 und 2C19) und [$^{14}$C]-Testosteron (CYP3A4) am Tag 8 bestimmt. Von den so gemessenen Enzymaktivitäten CYP1A2, 2B6, 2C19 und 3A4 behandelter Zellen im Vergleich zu unbehandelten Zellen wird das induktive Potential der Testsubstanzen ermittelt. Die folgende Tabelle 1 zeigt die Ergebnisse der Verbindung aus Beispiel 8 im Vergleich mit den Induktoren RIF. PB und OME:

Tabelle 1: induktiver Effekt auf Leberenzymaktivitäten in humanen Hepatozytenkulturen nach 8 Tagen Inkubation (normiert)

| | Konzentration | CYP1A2 | CYP2B6 | CYP2C19 | CYP3A4 |
|---|---|---|---|---|---|
| Kontrolle | 0 | 1,0 | 1,0 | 1,0 | 1,0 |
| RIF | 50 µM | n.b. | 5,15 | 15,21 | 9,15 |
| PB | 2000 µM | n.b. | 14,69 | 4,00 | 6,70 |
| OME | 100 µM | 28,57 | 1,54 | 1,61 | 1,45 |

(fortgesetzt)

| | Konzentration | CYP1A2 | CYP2B6 | CYP2C19 | CYP3A4 |
|---|---|---|---|---|---|
| Beispiel 8 | 0,01 µg/ml | 0,76 | 1,92 | 1,30 | 1,37 |
| | 0,1 µg/ml | 0,70 | 1,62 | 1,30 | 1,60 |
| | 1,0 µg/ml | 0,90 | 1,85 | 1,12 | 1,51 |
| | 10 µg/ml | 1,38 | 2,54 | 1,97 | 3,47 |
| n.b. = nicht bestimmt | | | | | |

Weiterer Gegenstand der vorliegenden Erfindung sind Arzneimittel, die die erfindungsgemäße Verbindung, vorzugsweise zusammen mit einem oder mehreren pharmakologisch unbedenklichen Hilfs- oder Trägerstoffen enthalten, sowie deren Verwendung zu den zuvor genannten Zwecken.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, topisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nicht überzogene sowie überzogene Tabletten, z.B. mit magensaftresistenten Überzüge versehene Tabletten oder Filmtabletten), Kapseln, Dragees, Granulate, Pellets, Pulver, Emulsionen, Suspensionen, Lösungen und Aerosole.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen / -lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate wie beispielsweise Stents.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien. Der Wirkstoff kann gegebenenfalls in einem oder mehreren der oben angegebenen Trägerstoffe auch in mikroverkapselter Form vorliegen.

Die therapeutisch wirksame Verbindung des Beispiels 8 soll in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer der erfindungsgemäßen Verbindung auch weitere pharmazeutische Wirkstoffe enthalten.

Im allgemeinen hat es sich sowohl in der Human- als auch in der Veterinärmedizin als vorteilhaft erwiesen, den erfindungsgemäßen Wirkstoff in Gesamtmengen von etwa 0,001 bis etwa 50, vorzugsweise 0,001 bis 10 mg/kg Körpergewicht je 24 Stunden, gegebenenfalls in Form mehrerer Einzelgaben, zur Erzielung der gewünschten Ergebnisse zu verabreichen. Eine Einzelgabe enthält den erfindungsgemäßen Wirkstoff vorzugsweise in Mengen von etwa 0,001 bis etwa 30, insbesondere 0,001 bis 3 mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente. Lösungsmittelverhältnisse, Verdünnungsverhältnisse und Konzentrationsangaben von flüssig/flüssig-Lösungen beziehen sich jeweils auf das Volumen.

**Abkürzungen:**

ACN Acetonitril

| | |
|---|---|
| BABA | n-Butylacetat/n-Butanol/Eisessig/Phosphatpuffer pH 6 (50:9:25.15; org. Phase) |
| DC | Dünnschichtchromatographie |
| DCI | direkte chemische Ionisation (bei MS) |
| DCM | Dichlormethan |
| DIEA | *N,N*-Düsopropylethylamin |
| DMSO | Dimethylsulfoxid |
| DMF | *N,N*-Dimethylformamid |
| d. Th. | der Theorie |
| EE | Ethylacetat (Essigsäureethylester) |
| EI | Elektronenstoß-Ionisation (bei MS) |
| ESI | Elektrospray-Ionisation (bei MS) |
| Fp. | Schmelzpunkt |
| ges. | gesättigt |
| h | Stunde |
| HPLC | Hochdruck-, Hochleistungsflüssigchromatographie |
| konz. | konzentriert |
| LC-MS | Flüssigchromatographie-gekoppelte Massenspektroskopie |
| LDA | Lithium-Diisopropylamid |
| MCPBA | m-Chlorperoxybenzoesäure |
| MS | Massenspektroskopie |
| NMR | Kernresonanzspektroskopie |
| proz. | prozentig |
| $R_f$ | Retentionsindex (bei DC) |
| RP-HPLC | Reverse Phase HPLC |
| RT | Raumtemperatur |
| $R_t$ | Retentionszeit (bei HPLC) |
| THF | Tetrahydrofuran |

**Laufmittel für die Dünnschichtehromatographie:**

| | |
|---|---|
| T1 E1 : | Toluol - Essigsäureethylester (1:1) |
| T1 EtOH1: | Toluol - Ethanol (1:1) |
| C1 E1 : | Cyclohexan - Essigsäureethylester (1:1) |
| C1 E2: | Cyclohexan - Essigsäureethylester (1:2) |

**LCMS- und HPLC-Methoden:**

Methode 1 (LCMS)

Instrument: Micromass Platform LCZ, HP1100; Säule: Symmetry C18, 50 mm x 2.1 mm, 3.5 μm; Eluent A: Acetonitril + 0.1% Ameisensäure, Eluent B: Wasser + 0.1 % Ameisensäure; Gradient: 0.0min 10 %A → 4.0min 90 %A → 6.0min 90 %A; Ofen: 40°C; Fluss: 0.5ml/min; UV-Detektion: 208-400 nm.

Methode 2 (LCMS)

Instrument: Micromass Quattro LCZ, HP1100; Säule: Symmetry C18, 50 mm x 2.1 mm, 3.5 μm; Eluent A: Acetonitril + 0.1 % Ameisensäure, Eluent B: Wasser + 0.1 % Ameisensäure; Gradient: 0.0min 10 %A → 4.0min 90 %A → 6.0min 90 %A; Ofen: 40°C; Fluss: 0.5ml/min; UV-Detektion: 208-400 nm.

Methode 3 (LCMS)

Instrument: Waters Alliance 2790 LC; Säule: Symmetry C18, 50mm x 2.1, 3.5μm; Eluent A: Wasser + 0.1 % Ameisensäure, Eluent B: Acetonitril + 0.1 % Ameisensäure; Gradient: 0.0min 5 % B → 5.0min 10 %B → 6.0min 10 %B; Temperatur: 50°C; Fluss: 1.0ml/min; UV-Detektion: 210nm.

Methode 4 (HPLC)

Instrument: HP 1100 mit DAD-Detektion; Säule: Kromasil RP-18, 60mm x 2mm, 3.5μm; Eluent: A=5ml $HClO_4$/l $H_2O$, B=ACN; Gradient: 0 min 2 %B, 0.5 min 2 %B, 4.5 min 90 %B, 6.5 min 90 %B; Fluß: 0.75 ml/min; Temp.: 30°C; Detektion UV 210 nm.

Präparative RP-HPLC

Säule: YMC-Gel; Eluent: Acetonitril/Wasser (Gradient); Fluß: 50 ml/min; Temp.: 25°C; Detektion UV 210 nm.

**Ausgangsverbindungen:**

**Beispiel 1A**

5-Amino-1-(2-fluorbenzyl)-pyrazol-3-carbonsäureethylester

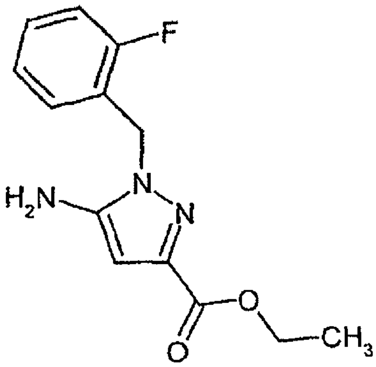

100 g (0.613 mol) Natriumsalz des Cyanobrenztraubensäureethylester (Darstellung analog Borsche und Manteuffel, Liebigs Ann. 1934, 512, 97) werden unter gutem Rühren unter Argon in 2.5 1 Dioxan bei Raumtemperatur mit 111.75 g (75 ml, 0.98 mol) Trifluoressigsäure versetzt und 10 Minuten gerührt, wobei ein großer Teil des Eduktes in Lösung geht. Dann gibt man 85.93 g (0.613 mol) 2-Fluorbenzylhydrazin hinzu und erhitzt über Nacht unter Rückfluss. Nach Abkühlen werden die ausgefallenen Kristalle des Natriunitrifluoracetats abgesaugt, mit Dioxan gewaschen und die Lösung wird roh weiter umgesetzt.

**Beispiel 2A**

1-(2-Fluorbenzyl)-1H-pyrazolo [3,4-b]pyridin-3-carbonsäureethylester

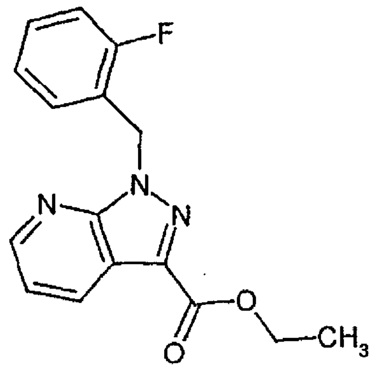

Die aus Beispiel 1A erhaltene Lösung wird mit 61.25 ml (60.77 g, 0.613 mol) Dimethylaminoacrolein und 56.28 ml

(83.88 g, 0.736 mol) Trifluoressigsäure versetzt und unter Argon 3 Tage lang gekocht. Anschließend wird das Lösungsmittel im Vakuum verdampft, der Rückstand in 21 Wasser gegeben und dreimal mit je 1 1 Essigsäureethylester extrahiert. Die vereinigten organischen Phasen werden mit Magnesiumsulfat getrocknet und am Rotationsverdampfer eingeengt. Man chromatographiert an 2.5 kg Kieselgel und eluiert mit einem Toluol / Toluol-Essigsäureethylester=4:1-Gradienten. Ausbeute: 91.6 g (49.9 % d.Th. über zwei Stufen).

Schmelzpunkt 85°C
$R_f$ ($SiO_2$, T1 E1): 0.83

**Beispiel 3A**

1-(2-Fluorbenzyl)-1 H-pyrazolo[3,4-b]pyridin-3-carboxamid

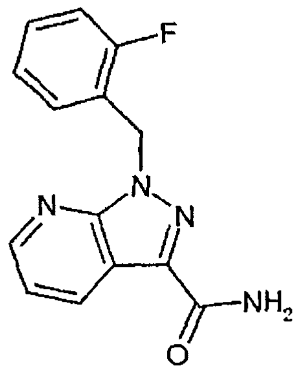

10.18 g (34 mmol) des in Beispiel 2A erhaltenen Esters werden in 150 ml Methanol, das mit Ammoniak bei 0 - 10°C gesättigt wurde, vorgelegt. Man rührt zwei Tage bei Raumtemperatur und engt anschließend im Vakuum ein. $R_f$ ($SiO_2$, T1 E1): 0.33

**Beispiel 4A**

3-Cyano-1-(2-fluorbenzyl)-1H-pyrazolo[3,4-b]pyridin

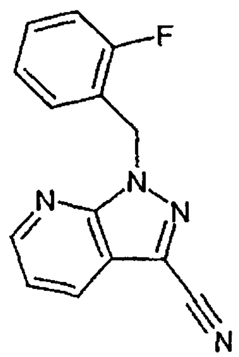

36.1 g (133 mmol) 1-(2-Fluorbenzyl)-1H-pyrazolo[3,4-b]pyridin-3-carboxamid aus Beispiel 3A werden in 330 ml THF gelöst und mit 27 g (341 mmol) Pyridin versetzt. Anschließend gibt man innerhalb von 10 Minuten 47.76 ml (71.66 g, 341 mmol) Trifluoressigsäureanhydrid hinzu, wobei die Temperatur bis auf 40°C ansteigt. Man rührt über Nacht bei Raumtemperatur. Anschließend wird der Ansatz in 11 Wasser gegeben und dreimal mit je 0.5 1 Essigsäureethylester extrahiert. Die organische Phase wird mit gesättigter Natriumhydrogencarbonatlösung und mit 1 N Salzsäure gewaschen, mit Magnesiumsulfat getrocknet und am Rotationsverdampfer eingeengt.

Ausbeute: 33.7 g (100 % d.Th.)

Schmelzpunkt: 81°C
$R_f$ ($SiO_2$, T1 E1): 0.74

**Beispiel 5A**

(2-Fluorbenzyl)-1H-pyrazolo[3,4-b]pyridin-3-carboaimidsäuremethylester

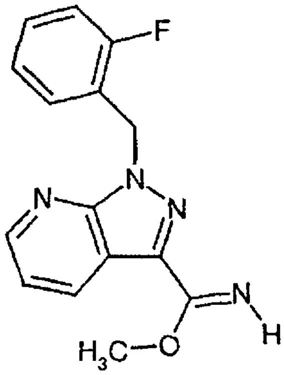

Man löst 30.37 g (562 mmol) Natriummethylat in 1.5 1 Methanol und gibt 36.45 g (144.5 mmol) 3-Cyano-1-(2-fluorbenzyl)-1H-pyrazolo[3,4-b]pyridin (aus Beispiel 4A) hinzu. Man rührt 2 Stunden bei Raumtemperatur und setzt die erhaltene Lösung direkt für die nächste Stufe ein.

**Beispiel 6A**

1-(2-Fluorbenzyl)1H-pyrazolo[3,4-b]pyridin-3-carboxamidin

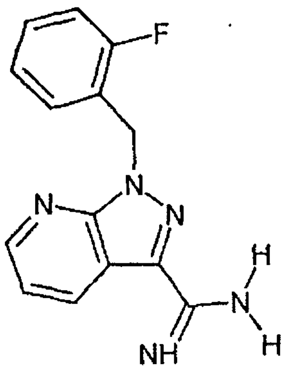

Die aus Beispiel 5A erhaltene Lösung von (2-Fluorbenzyl)-1H-pyrazolo[3,4-b]-pyridin-3-carboximidsäuremethylester in Methanol wird mit 33.76 g (32.19 ml, 562 mmol) Eisessig und 9.28 g (173 mmol) Ammoniumchlorid versetzt und über Nacht unter Rückfluss gerührt. Man verdampft das Lösungsmittel im Vakuum, verreibt den Rückstand gut mit Aceton und saugt den ausgefallenen Feststoff ab. Man gibt in 2 1 Wasser, versetzt unter Rühren mit 31.8 g Natriumcarbonat und extrahiert dreimal mit insgesamt 1 l Essigsäureethylester, trocknet die organische Phase mit Magnesiumsulfat und dampft im Vakuum ein.

Ausbeute 27.5 g (76.4 % d.Th. über zwei Stufen)
Smp.: 86°C
$R_f$($SiO_2$, T1 EtOH1): 0.08

**Beispiel 7A**

2-[1-(2-Fluorbenzyl)-1H-pyrazolo[3,4-b]pyridin-3-yl]-5-[(E)phenyldiazenyl]-4,6-pyrimidindiamin

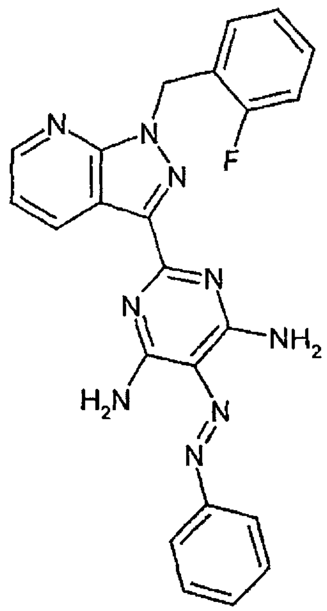

Man gibt zu einer gerührten Lösung von 21.92 g (71.7 mmol) 1-(2-Fluorbenzyl)1H-pyrazolo[3,4-b]pyridin-3-carboxamidin in N,N-Dimethylformamid aus Beispiel 6A 3.87 g Natriummethanolat und anschließend 12.2 g (71.7 mmol) Phenylazomalononitril (L. F. Cavalieri, J. F. Tanker, A. Bendich, J. Am. Chem. Soc., 1949, 71, 533). Man rührt über Nacht bei 110°C und lässt abkühlen. Der hierbei ausgefallene Feststoff wird abgesaugt und mit Ethanol gewaschen. Nach Trocknung erhält man 23 g (73 % d.Th.) der Zielverbindung.

**Beispiel 8A**

2-[1-(2-Fluorbenzyl)-1H-pyrazolo[3,4-b]pyridin-3-yl]-4,5,6-pyrimidintriamin Trihydrochlorid

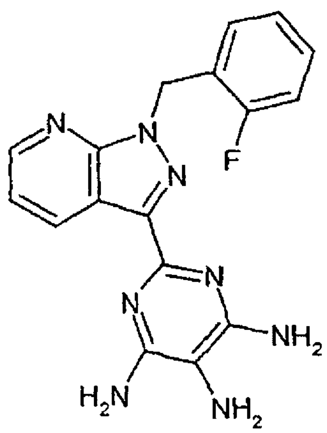

5 g (11.38 mmol) 2-[1-(2-Fluorbenzyl)-1H-pyrazolo[3,4-b]pyridin-3-yl]-5-[(E)-phenyldiazenyl]-4,6-pyrimidindiamin aus Beispiel 7A werden mit 800 mg 50 proz. Raney-Nickel in Wasser in 60 ml DMF 22 Stunden lang bei 65 bar Wasserstoffdruck und 62°C hydriert. Man saugt vom Katalysator über Kieselguhr ab, dampft die Lösung im Vakuum ein und rührt mit 5 N Salzsäure. Der ausgefallene gelbbraune Niederschlag wird abgesaugt und getrocknet. Man erhält 3.1 g (59.3 % d. Th.) der Zielverbindung. Die freie Base erhält man durch Ausschütteln mit verdünnter Natriumhydrogencarbonat-Lösung und Extrahieren mit Essigsäureethylester. Der in beiden Phasen unlösliche Feststoff wird abgesaugt. Auch die Essigsäureethyl-esterphase enthält geringe Mengen der freien Base.

**Beispiel 9A**

Methylcyanomethyl(methyl)carbamat

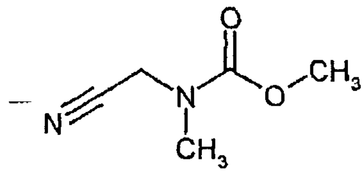

Herstellung analog: Q. Li. Chu, T.W. Daniel, A. Claiborne, C.S. Cooper, C.M. Lee, J. Med. Chem.1996, 39, 3070-3088.

**Beispiel 10A**

Natrium-(E)-2-cyano-2-[(methoxycarbonyl)(methyl)amino]ethenolat

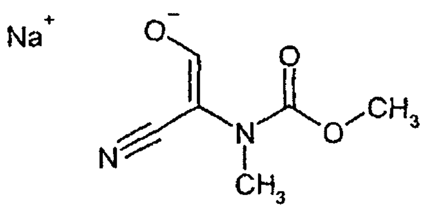

Unter Argon werden0.46g (0.01 mmol) Natriummethylat in Tetrahydrofuran gegeben (Lösung A). Anschließend gibt man 1.00 g (0.01 mmol) Methylcyanomethyl(methyl)carbamat aus Beispiel 9A in 1.73 g (0.02 mmol) Ameisensäureethylester. Zu dieser Mischung wird Lösung A langsam zugetropft. Es wird über Nacht bei RT gerührt. Das Lösungsmittel wird in Vakuum am Rotationsverdamper eingeengt und der Rückstand wird mit Diethylether versetzt. Die ausgefallenen Kristalle werden abgesaugt und im Hochvakuum getrocknet.

Ausbeute: 1.05 g (76 % d. Th.)
HPLC (Methode 4): $R_t$= 1.3 min.
$^1$H-NMR (200 MHz, DMSO-$d_6$): $\delta$ = 2.90 (d, 1H), 3.35 (s, 3H), 3.47 (s, 3H).

**Beispiele**

**Beispiel 1** (Bezugsbeispiel)

Ethyl-4-amino-2-[1-(2-fluorbenzyl)-1H-pyrazolo[3,4-b]pyridin-3-yl]-5-pyrimidinyl-(methyl)carbamat

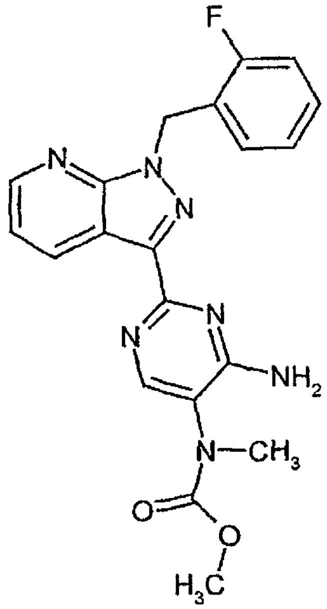

Man gibt unter Argon 0.80 g (2.61 mmol) 1-(2-Fluorbenzyl)1H-pyrazolo[3,4-b]pyridin-3-carboxamidin aus Beispiel 6A, 0.51 g (2.86 mmol) Natrium-(E)-2-cyano-2-[(methoxycarbonyl)(methyl)amino]ethenolat aus Beispiel 10A und 0.53 g 0.73 ml (5.23 mmol) Triethylamin in 50 ml Toluol. Es wird 9 Stunden unter Rückfluss erhitzt. Anschließend wird wieder auf RT abgekühlt, mit Dichlormethan und Wasser versetzt und extrahiert. Die organische Phase wird über Magnesiumsulfat getrocknet, abfiltriert und im Vakuum am Rotationsverdampfer eingeengt. Der Rückstand wird mit 5 ml Diethylether versetzt und kristallisiert dabei aus. Die Kristalle werden abgesaugt, getrocknet und über präparative RP-HPLC gereinigt.

Ausbeute: 20.2 mg (2 % d. Th.)
LC/MS (Methode 2): Rt = 3.01 min
MS (EI): m/z = 408 $(M+H)^+$
$^1$H-NMR (300 MHz, DMSO-$d_6$): $\delta$= 3.09 (s, 3H), 3.29 (s, 3H), 5.83 (s, 2H), 7.09-7.42 (m, 5H), 8.20 (s, 1H), 8.64 (dd, 1H). 8.94 (dd, 1H), 9.27 (br. s, 2H).

**Beispiel 2** (Bezugsbeispiel)

Ethyl-4,6-diamino-2-[1-(2-fluorbenzyl)-1H-pyrazolo[3,4-b]pyridin-3-yl]-5-pyrimidinylcarbamat

Man gibt 107.35 mg (0.31 mmol) 2-[1-(2-Fluorbenzyl)-1H-pyrazolo[3,4-b]pyridin-3-yl]-4,5,6-pyrimidintriamin Trihydrochlorid aus Beispiel 8A in 5ml Pyridin und kühlt die Mischung auf 0°C ab. Dazu gibt man 33.25 mg (0.31 mmol) Chlorameisensäureethylester und lässt die Reaktion über Nacht bei RT rühren. Das Pyridin wird im Vakuum einrotiert, und der Rückstand wird über präparative RP-HPLC gereinigt.

Ausbeute: 56.2 mg (43 % d. Th.)
LC/MS (Methode 1): $R_t$= 2.66 min
MS (EI): m/z = 423 $(M+H)^+$
$^1$H-NMR (300 MHz, DMSO-$d_6$): $\delta$ = 1.17-1.33 (m, 3H), 3.97-4.14 (m, 2H), 5.80 (s, 2H), 6.14 (br. s, 4H), 7.07-7.17 (m, 2H), 7.22 (t, 1H). 7.29-7.40 (m, 2H), 7.97 (br. s, 1H), 8.60 (d, 1H), 9.07 (d, 1H).

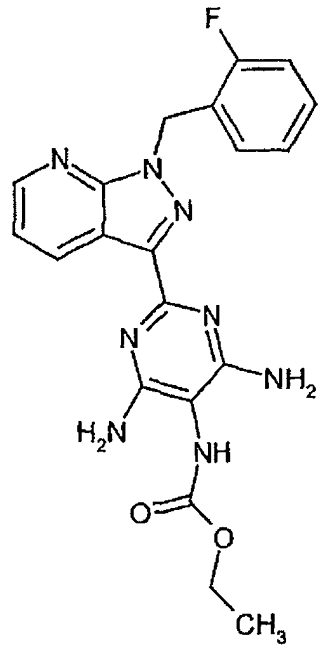

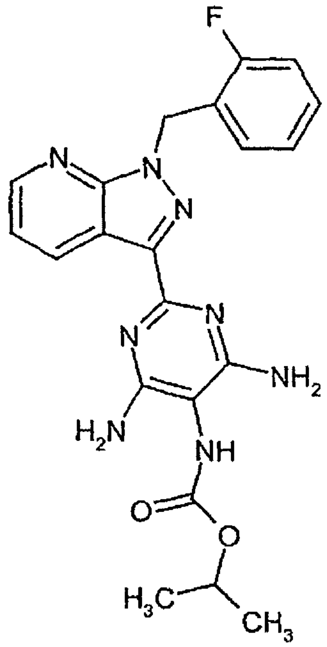

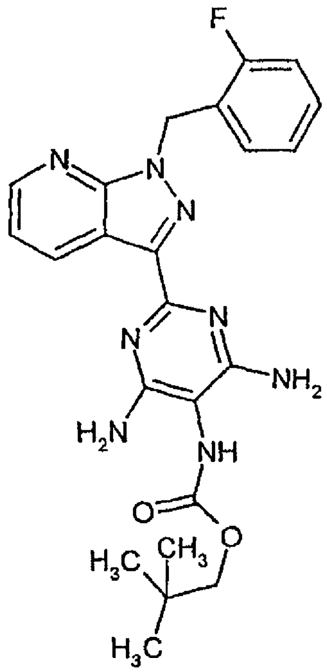

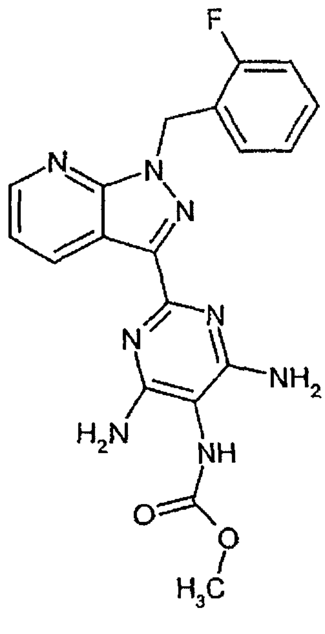

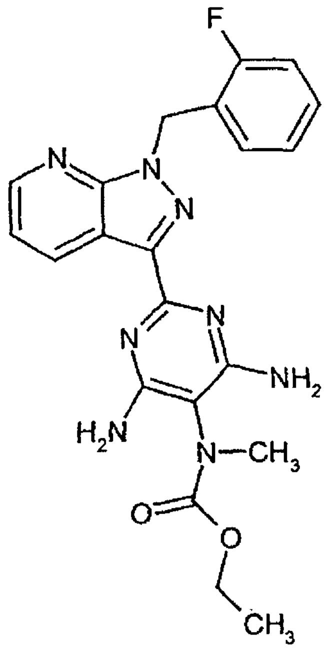

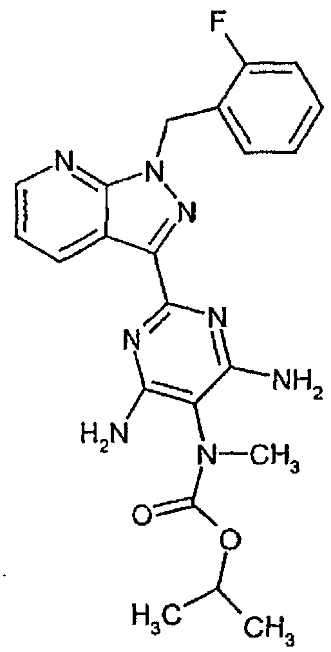

Herstellung analog Beispiel 2 mit 150 mg (0.43 mmol) 2-[1-(2-Fluorbenzyl)-1H-pyrazolo[3,4-b]pyridin-3-yl]-4,5,6-pyrimidintriamin Trihydrochlorid aus Beispiel 8A, 7.5 ml Pyridin und 52.47 mg (0.43 mmol) Isopropylchloroformat. Der Rückstand wird in einem Dichlormethan/Methanol Gemisch aufgenommen, abfiltriert und getrocknet.

Ausbeute: 165 mg (88 % d. Th.)
LC/MS (Methode 1): Rt = 2.84 min
MS (EI): m/z = 437 $(M+H)^+$
$^1$H-NMR (300 MHz, DMSO-$d_6$): δ = 1.26 (d, 6H), 4.82 (quin., 1H), 5.92 (s, 2H), 7.07-7.20 (m, 2H), 7.25 (t, 1H). 7.31-7.43 (m, 2H), 7.47-7.57 (m, 1H), 8.16 (br. s, 1H), 8.74 (dd, 1H), 8.98 (dd, 1H).

**Beispiel 4** (Bezugsbeispiel)

Neopentyl-4,6-diamino-2-[1-(2-fluorbenzyl)-1H-pyrazolo[3,4-b]pyridin-3-yl]-5-pyrimidinylcarbamat

Herstellung analog Beispiel 2 mit 100 mg (0.29 mmol) 2-[1-(2-Fluorbenzyl)-1H-pyrazolo[3,4-b]pyridin-3-yl]-4,5,6-pyrimidintriamin Trihydrochlorid aus Beispiel 8A, 5 ml Pyridin und 43 mg (0.29 mmol) Neopentylchloridocarbonat.

Ausbeute: 54 mg (41 % d. Th.)
LC/MS (Methode 1): Rt = 3.10 min
MS (EI): m/z = 465 $(M+H)^+$
$^1$H-NMR (400 MHz, DMSO-$d_6$): $\delta$= 0.95 (br. s, 9H), 3.74 (s, 2H), 5.79 (s, 2H), 6.10 (br. s, 4H), 7.08-7.17 (m, 2H). 7.22 (t, 1H), 7.29-7.39 (m, 2H), 8.00 (br. s, 1H), 8.60 (dd, 1H), 9.06 (dd, 1H).

**Beispiel 5** (Bezugsbeispiel)

Methyl-4,6-diamino-2-[1-(2-fluorbenzyl)-1H-pyrazolo[3,4-b]pyridin-3-yl]-5-pyrimidinylcarbamat

30.5 g (87.0 mmol) 2-[1-(2-Fluorbenzyl)-1H-pyrazolo[3,-b]pyridin-3-yl]-4,5,6-pyrimidintriamin Trihydrochlorid aus Beispiel 8A werden in 30 ml Pyridin gelöst. Die entstehende Lösung wird auf 0°C gekühlt. Man versetzt mit 8.22 g (87.0 mmol) Chlorameisensäuremethylester und rührt weitere 2 Stunden bei 0°C. Anschließend lässt man auf Raumtemperatur erwärmen und rührt für weitere 12 Stunden. Nach Einengen im Vakuum wird der Rückstand mit Wasser gewaschen und getrocknet. Zur weiteren Reinigung wird in 300 ml siedendem Diethylether ausgerührt. Das ausgefallene Produkt wird abgesaugt und im Vakuum getrocknet.

Ausbeute: 32.6 g (92 % d. Th.)
LC/MS (Methode 1): Rt = 2.61 min
MS (EI): m/z = 409 $(M+H)^+$
$^1$H-NMR (400 MHz, "DMSO-$d_6$): $\delta$= 3.61 (s, 3R), 5.80 (s, 2H), 6.19 (br. s, 4H), 7.08-7.16 (m, 2H). 7.22 (t, 1H), 7.28-7.39 (m, 2H), 7.99 (br. s, 1H), 8.60 (dd, 1H), 9.05 (dd, 1H).

**Beispiel 6** (Bezugsbeispiel)

Ethyl-4,6-diamino-2-[1-(2-ftuorbenzyl)-1H-pyrazolo[3,4-b]pyridin-3-yl]-5-pyrimidinyl(methyl)carbamat

Man gibt 54 mg (0.13 mmol) Ethyl-4,6-diamino-2-[1-(2-fluorbenzyl)-1H-pyrazolo-[3,4-b]pyridin-3-yl]-5-pyrimidinylcarbamat aus Beispiel 3 in 5 ml DMF, kühlt die Mischung auf 0°C ab und versetzt mit 7.67 mg (0.19 mmol) Natriumhydrid. Anschließend tropft man 18.14 mg (0.13 mmol) lodmethan dazu und rührt eine Stunde nach. Man versetzt das Gemisch mit Wasser und extrahiert mit Dichlormethan. Die vereinigten organischen Phasen werden über Magnesiumsulfat getrocknet und am Rotationsverdampfer eingeengt. Der Rückstand wird zuerst säulenchromatographisch (Laufmittel: Dichlormethan/Methanol = 10:1) und anschließend über präparative RP-HPLC gereinigt.

Ausbeute: 32 mg (58 % d. Th.)
LC/MS (Methode 2): Rt = 2.91 min
MS (EI): m/z = 437 $(M+H)^+$
$^1$H-NMR (200 MHz, DMSO-$d_6$): $\delta$= 1.08 (t, 3H), 2.99 (s, 3H), 2.93-4.11 (m, 2H), 5.79 (s, 2H), 6.35 (br. s, 4H), 7.06-7.14 (m, 2H), 7.16-7.28 (m, 1H), 7.28-7.32 (m, 2H). 8.59 (dd, 1H), 9.06 (dd, 1H).

**Beispiel 7** (Bezugsbeispiel)

Isopropyl-4,6-diamino-2-[1-(2-fluorbenzyl)-1H-pyrazolo[3,4-b]pyridin-3-yl]-5-pyrimidinyl(methyl)carbamat

Herstellung analog Beispiel 6 mit 75 mg (0.17 mmol) Isopropyl-4,6-diamino-2-[1-(2-fluorbenzyl)-1H-pyrazolo [3,4-b]pyridin-3-yl]-5-pyrimidinylcarbamat aus Beispiel 3, 10.31 mg (0.26 mmol) Natriumhydrid und 24.4 mg (0.17 mmol) lodmethan. Der Rückstand wird über präparative RP-HPLC gereinigt.

Ausbeute: 32 mg (41 % d. Th.)
LCIMS (Methode 1): $R_t$ = 2.97 min
MS (EI): m/z = 451 $(M+H)^+$
$^1$H-NMR (300 MHz, DMSO-$d_6$): $\delta$ = 1.09 (d, 6H), 2.98 (s, 3H), 4.80 (quin., 1H), 5.79 (s, 2H), 6.31 (br. s, 4H), 7.05-7.16 (m, 2H), 7.22 (t, 1H), 7.28-7.40 (m, 2H), 8.59 (dd, 1H), 9.07 (dd, 1H).

**Beispiel 8**

Methyl-4,6-diamino-2-[1-(2-fluorbenzyl)-1H-pyrazolo[3,4-b]pyridin-3-yl]-5-pyrimidinyl(methyl)carbamat

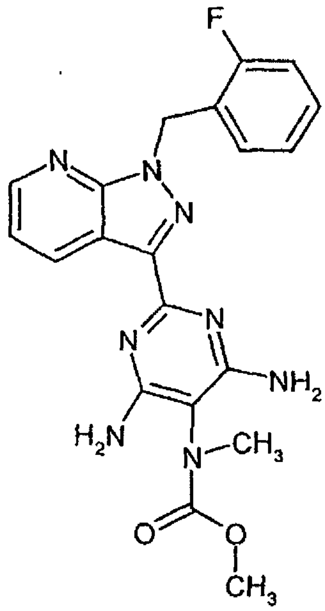

Herstellung analog Beispiel 6 mit 310 mg (0.76 mmol) Methyl-4,6-diamino-2-[1-(2-fluorbenzyl)-1H-pyrazolo [3,4-b]pyridin-3-yl]-5-pyrimidinylcarbamat aus Beispiel 5, 27.32 mg (1.14 mmol) Natriumhydrid und 215.5 mg (1.52 mmol) Iodmethan. Zur Aufarbeitung versetzt man das Gemisch mit Wasser und 2 molarer Kaliumhydroxid-Lösung und extrahiert mit Dichlormethan Die vereinigten organischen Phasen werden mit Magnesiumsulfat getrocknet und am Rotationsverdampfer eingeengt. Der Rückstand wird über präparative RP-HPLC gereinigt.
Ausbeute: 93 mg (29 % d. Th.)

Größere Mengen der Verbindung aus Beispiel 8 können auch nach folgender Synthesevorschrift hergestellt werden:
20.0 g (49.0 mmol) Methyl-4,6-diamino-2-(1-(2-fluorbenzyl)-1H-pyrazolo[3,4-b]-pyridin-3-yl]-5-pyrimidinylcarbamat aus Beispiel 5 werden in 257 ml Tetrahydrofuran gelöst und auf 0°C gekühlt. 53.9 ml (49.0 mmol einer 1 M Lösung in Tetrahydrofuran) Bis-(trimethylsilyl)-lithiumamid werden innerhalb von 15 Minuten zugetropft. Man rührt 20 min bei 0°C nach und versetzt dann mit 6.95 g (53.9 mmol) Iodmethan. Nach einer Stunde lässt man auf Raumtemperatur erwärmen und beendet die Reaktion durch Zugabe von gesättigter wässriger Ammoniumchlorid-Lösung. Die Phasen werden getrennt. Die wässrige Phase wird mehrfach mit Essigsäureethylester und Dichlormethan extrahiert. Die vereinigten organischen Phasen werden im Vakuum eingeengt. Der so erhaltene Rückstand wird in einer Mischung aus Dichlormethan und Tetrahydrofuran (1:1) suspendiert. Die unlöslichen Kristalle werden abgesaugt und in Methanol aufgenommen. Man erhitzt für eine Stunde unter Rückfluss. Nach dem Abkühlen filtriert man vom ausgefallenen Niederschlag ab. Der so erhaltene rote Feststoff wird in 100 ml einer Mischung aus Dioxan und Dichlormethan (1:1) suspendiert und in der Siedehitze mit 20 ml Methanol versetzt bis sich eine klare Lösung bildet. Man versetzt mit Aktivkohle, kocht kurz auf und filtriert heiß über Kieselgur. Die so erhaltene Lösung wird zur Trockene eingeengt. Man nimmt in Methanol auf und rührt die Suspension für eine Stunde bei Raumtemperatur Die weißen Kristalle werden abgesaugt.

Ausbeute: 14.9 g (72 % d. Th.)
LC/MS (Methode 3): $R_t$ =1.85 min
MS (EI): m/z = 423 $(M+H)^+$
$^1$H-NMR (200 MHz, DMSO-$d_6$): $\delta$ = 3.01 (s, 3H), 3.57 (s, 3H), 5.92 (s, 2H), 7.05.7.17 (m, 2H), 7.18-7.46 (m, 3H), 7.47-7.61 (m, 2H), 7.59-7.97 (m, 2H), 8.71-8.81 (m, 1H), 8.97 (dd, 1H).

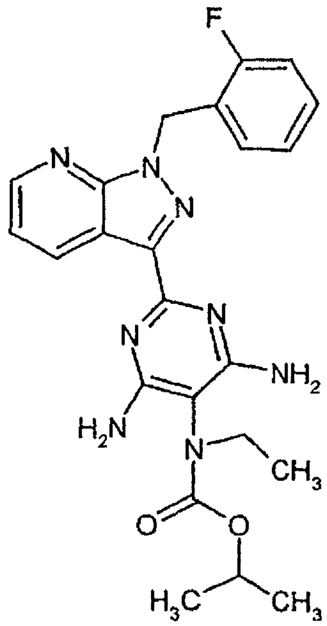

Herstellung analog Beispiel 6 mit 60 mg (0.14 mmol) Isopropyl-4,6-diamino-2-[1-(2-fluorbenzyl)-1H-pyrazolo [3,4-b]pyridin-3-yl]-5-pyrimidinylcarbamat aus Beispiel 3, 4.95 mg (0.21 mmol) Natriumhydrid und 21.4 mg (0.17 mmol) lodethan. Zur vollständigen Umsetzung werden noch einmal die gleiche Menge Natriumhydrid und lodethan dazugegeben. Der Rückstand wird über präparative RP-HPLC gereinigt.

Ausbeute: 43 mg (67 % d. Th.)
LC/MS (Methode 1): Rt = 2.97 min
MS (EI): m/z = 465 $(M+H)^+$
$^1$H-NMR (200 MHz, DMSO-$d_6$): $\delta$ = 0.96-1.06 (m, 3H), 1.09 (d, 6H), 2.79-2.93 (m, 2H), 4.82 (quin., 1H), 5.80 (s, 2H), 6.25 (br. s, 4H), 7.01-7.14 (m, 2H), 7.15-7.50 (m, 3H), 8.60 (dd, 1H), 9.09 (dd, 1H).

## Patentansprüche

**1.** Verbindung mit folgender Struktur:

Methyl-4,6-diamino-2-[1-(2-fluorbenzyl)-1H-pyrazolo[3,4-b]pyridin-3-yl]-5-pyrimidinyl(methyl)carbamat

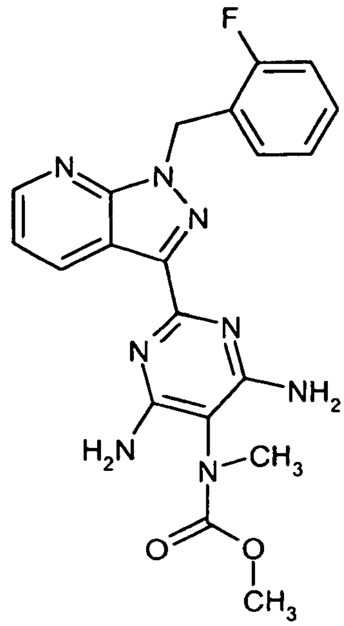

sowie ihre Salze, Isomere und Hydrate.

**2.** Verfahren zur Herstellung der Verbindung wie in Anspruch 1 definiert, **dadurch gekennzeichnet, dass** man

[A] Verbindungen der Formel (Ia)

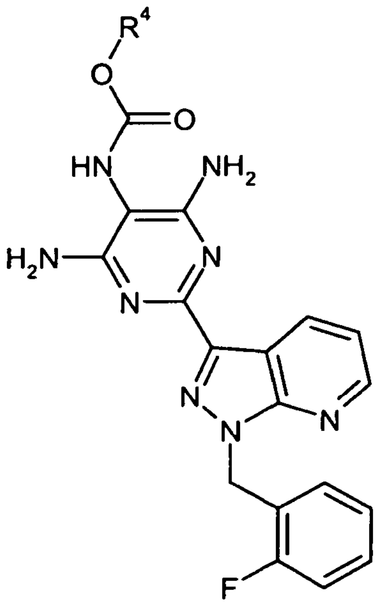

(Ia),

worin

$R^4$ Methyl ist,
mit Verbindungen der Formel (II)

$R^3$-$X^1$ (II),

worin
$R^3$ Methyl ist,
und

$X^1$ für eine Abgangsgruppe steht,
umsetzt.

**3.** Verbindung, wie in Anspruch 1 definiert, zur Behandlung von Erkrankungen.

**4.** Arzneimittel, enthaltend mindestens die Verbindung, wie in Anspruch 1 definiert, und mindestens einen weiteren Hilfsstoff.

**5.** Arzneimittel enthaltend mindestens die Verbindung, wie in Anspruch 1 definiert, in Kombination mit mindestens einem organischen Nitrat oder NO-Donator.

**6.** Arzneimittel enthaltend mindestens die Verbindung, wie in Anspruch 1 definiert, in Kombination mit mindestens einer Verbindung, die den Abbau von cyclischem Guanosinmonophosphat (cGMP) inhibiert.

**7.** Verwendung der Verbindung, wie in Anspruch 1 definiert, zur Herstellung von Arzneimitteln zur Behandlung von Herz-Kreislauf-Erkrankungen.

**8.** Verwendung der Verbindung, wie in Anspruch 1 definiert, zur Herstellung von Arzneimitteln zur Behandlung von Hypertonie.

**9.** Verwendung der Verbindung, wie in Anspruch 1 definiert, zur Herstellung von Arzneimitteln zur Behandlung von thromboembolischen Erkrankungen und Ischämien.

**10.** Verwendung der Verbindung, wie in Anspruch 1 definiert, zur Herstellung von Arzneimitteln zur Behandlung von sexueller Dysfunktion, insbesondere von erektiler Dysfunktion und von weiblicher sexueller Dysfunktion.

**11.** Verwendung gemäß einem der Ansprüche 7 bis 10, wobei die Verbindung, wie in Anspruch 1 definiert, in Kombination mit mindestens einem organischen Nitrat oder NO-Donator oder in Kombination mit mindestens einer Verbindung, die den Abbau von cyclischem Guanosinmonophosphat (cGMP) inhibiert, eingesetzt wird.

**Claims**

**1.** Compound with the following structure:
Methyl-4,6-diamino-2-[1-(2-fluorobenzyl)-1H-pyrazolo[3,4-b]pyridine-3-yl]-5-pyrimidinyl(methyl)carbamate

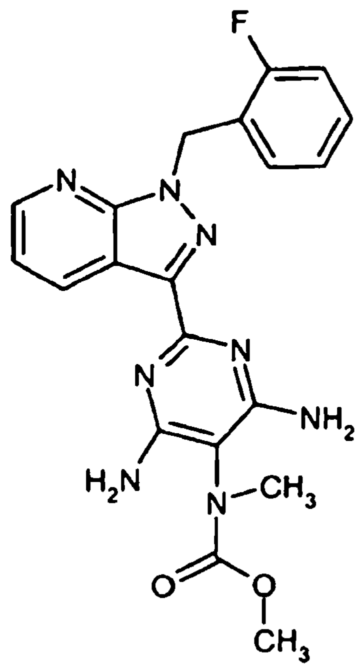

as well as its salts, isomers and hydrates.

2. Method for producing the compound as defined in claim 1, **characterised in that** [A] compounds of the formula (Ia)

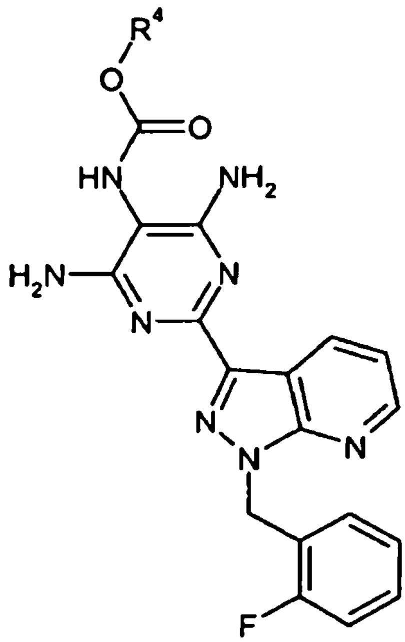

(Ia),

wherein

$R^4$ is methyl,
are reacted with compounds of the formula (II)

$R^3$-$X^1$ (II),

wherein
$R^3$ is methyl,
and
$X^1$ stands for a leaving group.

3. Compound as defined in claim 1 for the treatment of diseases.

4. Pharmaceutical product containing at least the compound as defined in claim 1 and at least one other excipient.

5. Pharmaceutical product containing at least the compound as defined in claim 1 in combination with at least one organic nitrate or NO donor.

6. Pharmaceutical product containing at least the compound as defined in claim 1 in combination with at least one compound which inhibits the degradation of cyclic guanosine monophosphate (cGMP).

7. Use of the compound as defined in claim 1 for the preparation of pharmaceutical products for the treatment of cardiovascular diseases.

8. Use of the compound as defined in claim 1 for the preparation of pharmaceutical products for the treatment of hypertension.

9. Use of the compound as defined in claim 1 for the preparation of pharmaceutical products for the treatment of thromboembolic diseases and ischemia.

10. Use of the compound as defined in claim 1 for the preparation of pharmaceutical products for the treatment of sexual

dysfunction, in particular erectile dysfunction and female sexual dysfunction.

**11.** Use according to one of the claims 7 to 10, wherein the compound as defined in claim 1 is used in combination with at least one organic nitrate or NO donor or in combination with at least one compound which inhibits the degradation of cyclic guanosine monophosphate (cGMP).

**Revendications**

**1.** Composé avec la structure suivante : méthyl-4,6-diamino-2-[1-(2-fluorobenzyle)-1H-pyrazolo[3,4-b]pyridine-3-yl]-5-pyrimidinyl(méthyl)carbamate

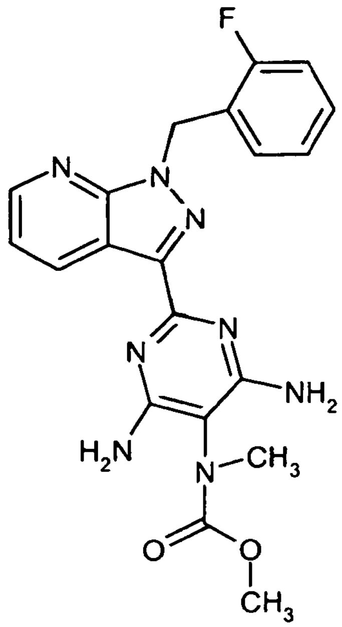

ainsi que ses sels, isomères et hydrates.

**2.** Procédé de réalisation du composé tel que défini dans la revendication 1, **caractérisé en ce qu'**on met en réaction

[A] des composés de la formule (Ia)

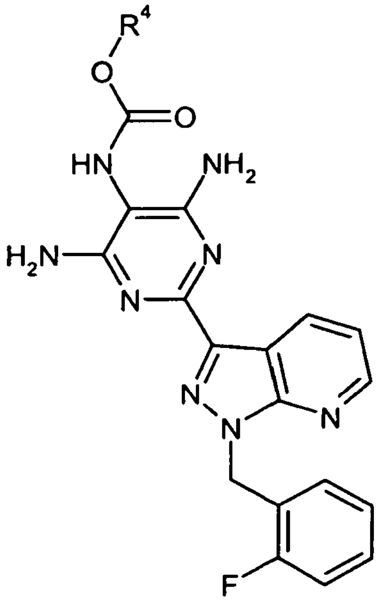

(Ia),

dans laquelle

$R^4$ est du méthyle,
avec des composés de la formule (II)

$R^3$-$X^1$ (II),

dans laquelle
$R^3$ est du méthyle,
et
$X^1$ représente un groupe de départ.

**3.** Composé tel que défini dans la revendication 1 pour le traitement de maladies.

**4.** Médicament contenant au moins le composé tel que défini dans la revendication 1 et au moins un excipient supplémentaire.

**5.** Médicament contenant au moins le composé tel que défini dans la revendication 1, en combinaison avec au moins un nitrate organique ou un donneur de NO.

**6.** Médicament contenant au moins le composé tel que défini dans la revendication 1, en combinaison avec au moins un composé, qui inhibe la dégradation de la guanosine monophosphate cyclique (GMPc).

**7.** Utilisation du composé tel que défini dans la revendication 1 pour la fabrication de médicaments destinés au traitement de maladies cardiovasculaires.

**8.** Utilisation du composé tel que défini dans la revendication 1, pour la fabrication de médicaments destinés au traitement de l'hypertension.

**9.** Utilisation du composé tel que défini dans la revendication 1, pour la fabrication de médicaments destinés au traitement de maladies thrombo-emboliques et d'ischémies.

**10.** Utilisation du composé tel que défini dans la revendication 1, pour la fabrication de médicaments pour le traitement de dysfonction sexuelle, en particulier de la dysfonction érectile et de la dysfonction sexuelle chez la femme.

**11.** Utilisation selon l'une quelconque des revendications 7 à 10, dans laquelle le composé tel que défini dans la revendication 1 est utilisé en combinaison avec au moins un nitrate organique ou un donneur de NO ou en

combinaison avec au moins un composé, qui inhibe la dégradation de la guanosine monophosphate cyclique (GMPc).

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9816223 A
- WO 9816507 A
- WO 9823619 A
- WO 0006567 A
- WO 0006568 A
- WO 0006569 A
- WO 0021954 A
- WO 0242299 A
- WO 0242300 A
- WO 0242301 A
- WO 0242302 A
- WO 02092596 A
- WO 03004503 A
- EP 0463756 A
- WO 9428902 A
- WO 9924433 A
- WO 9519978 A


### In der Beschreibung aufgeführte Nicht-Patentliteratur


- **WU et al.** *Blood 84*, 1994, 4226
- **MÜLSCH et al.** *Brit. J. Pharmacol.*, 1997, vol. 120, 681
- **GOLDBERG et al.** *J. Biol. Chem.*, 1977, vol. 252, 1279
- **PETTIBONE et al.** *Eur. J. Pharmacol.*, 1985, vol. 116, 307
- **YU et al.** *Brit. J. Pharmacol.*, 1995, vol. 114, 1587
- **L. F. CAVALIERI** ; **J. F. TANKER** ; **A. BENDICH**. Die Verbindung (VII) kann analog. *J. Am. Chem. Soc.*, 1949, vol. 71, 533
- **BORSCHE** ; **MANTEUFFEL**. *Liebigs. Ann. Chem.*, 1934, vol. 512, 97
- **Q. LI. CHU** ; **T. W. DANIEL** ; **A. CLAIBOME** ; **C. S. COOPER** ; **C. M. LEE**. *J. Med. Chem.*, 1996, vol. 39, 3070-3088
- **NOMENKLATUR NACH BEAVO** ; **REIFSNYDER**. *TiPS*, 1990, vol. 11, 150-155
- **E. BISCHOFF** ; **K. SCHNEIDER**. *Int. J. of Impotence Res.*, 2001, vol. 13, 230-235
- **E. BISCHOFF** ; **U. NIEWOEHNER** ; **H. HANING** ; **M. ES SAYED** ; **T. SCHENKE** ; **K. H. SCHLEMMER**. *The Journal of Urology*, 2001, vol. 165, 1316-1318
- **E. BISCHOFF**. *Int. J. Impotence Res.*, 2001, vol. 13, 146-148
- *Anal. Biochem.*, 1997, vol. 248, 188
- Darstellung analog Borsche und Manteuffel. *Liebigs Ann.*, 1934, vol. 512, 97
- **L. F. CAVALIERI** ; **J. F. TANKER** ; **A. BENDICH**. *J. Am. Chem. Soc.*, 1949, vol. 71, 533
- **Q. LI. CHU** ; **T.W. DANIEL** ; **A. CLAIBORNE** ; **C.S. COOPER** ; **C.M. LEE**. *J. Med. Chem.*, 1996, vol. 39, 3070-3088